# EUROPÄISCHE PATENTSCHRIFT

(11) **EP 0 917 566 B1**
(45) Veröffentlichungstag und Bekanntmachung des Hinweises auf die Patenterteilung: **22.09.2004**
(21) Anmeldenummer: 97927169.9
(22) Anmeldetag: 11.06.1997
(51) Int. Cl.: C12N 9/64, C07K 14/745

(54) **REKOMBINANTE BLUTGERINNUNGSPROTEASEN**
RECOMBINANT BLOOD-COAGULATION PROTEASES
PROTEASES RECOMBINEES DE COAGULATION SANGUINE

(30) Priorität: 11.06.1996 EP 96109288; 22.06.1996 EP 96110109; 06.07.1996 EP 96110959
(43) Veröffentlichungstag der Anmeldung: 26.05.1999
(73) Patentinhaber: Roche Diagnostics GmbH, 68305 Mannheim (DE)
(72) Erfinder: KOPETZKI, Erhard, D-82377 Penzberg (DE); HOPFNER, Karl-Peter, D-81673 München (DE)
(86) Internationale Anmeldenummer: PCT/EP1997/003027
(87) Internationale Veröffentlichungsnummer: WO 1997/047737

(56) Entgegenhaltungen:
- WO-A-94/18227
- PROTEIN ENGINEERING, Bd. 7, Nr. 9, 1994, Seiten 1121-1127, XP000605412 P.E. HUGUES ET AL.: "Protein engineering of the hydrophobic domain of human factor IX"
- JOURNAL OF BIOLOGICAL CHEMISTRY, Bd. 264, Nr. 24, 1989, Seiten 14298-14304, XP000051837 H.J. EHRLICH ET AL.: "Direct expression of recombinant activated human protein C, a serum protease"
- JOURNAL OF CLINICAL INVESTIGATION, Bd. 80, Nr. 4, 1987, Seiten 1023-1028, XP000605756 B.G. SCHACH ET AL.: "Hemophilia B due to a single nucleotide deletion in the gene for Factor IX"

## Beschreibung

Gegenstand der Erfindung sind verkürzte posttranslational nicht modifizierte Blutplasmaproteasevarianten der Faktor IX Genfamilie (FVII, FIX, FX und Protein C) bestehend aus EGF2-Domäne, Aktivierungspeptid (AP) und katalytischer Domäne (CD) sowie Verfahren zu ihrer Herstellung durch Expression in einer Wirtszelle, vorzugsweise in einem Mikroorganismus, Naturierung in vitro und anschließende Aktivierung mit einer geeigneten Protease.

Die erfindungsgemäßen Blutplasmaproteasevarianten sind geeignet zum Auffinden (Sreening) von Inhibitoren, zur Herstellung von Co-Kristallen aus Proteasevariante und Inhibitor zwecks Röntgenstrukturanalyse und "drug modelling" und als diagnostische Testkomponente in Aktivatortests.

Blutplasmaproteasen spielen sowohl eine Rolle bei der Blutgerinnung, dem Wundverschluß durch Fibrinbildung als auch bei der Fibrinolyse, der Clotauflösung bei der Wundheilung. Nach einer Verletzung wird durch sequentielle Aktivierung (spezifische Proteolyse) von inaktiven Proenzymen zu aktiven Enzymen das "Verletzungssignal" amplifiziert, wodurch die Blutgerinnung eingeleitet und ein schneller Wundverschluß gewährleistet wird. Die Blutgerinnung kann über zwei Wege initiiert werden, dem intrinsischen Weg, bei dem alle Proteinkomponenten im Blut vorhanden sind, und dem extrinsischen Weg, bei dem ein Membranprotein, der sogenannte "tissue factor", eine kritische Rolle spielt.

Der molekulare Mechanismus der Bluthomöostase (Blutgerinnung, Fibrinolyse und die Regulation dieses Gleichgewichts) und der daran beteiligten Komponenten ist in einigen Übersichtsartikeln umfassend dargestellt worden (Furie, B. und Furie, B.C., Cell 53 (1988) 505-518; Davie, E.W. et al. Biochem. 30 (1991) 10363-10379; Bergmeyer, H.U. (ed.), Methods of Enzymatic Analysis, Vol. V, chapter 3, 3rd ed., Academic Press, New York (1983)).

Die Proteasen der Blutgerinnungskaskade sind sehr komplexe Proteine. Sie können in der Regel nur mit großem Aufwand in begrenzter Menge, mit schwankender Qualität, Homogenität und Reinheit aus der natürlichen Rohstoffquelle, dem Blutplasma, isoliert werden (Van Dam-Mieras, M.C.E. et al., In: Bergmeyer, H.U. (ed.), Methods of Enzymatic Analysis, Vol. V, 3rd ed., page 365-394, Academic Press, New York (1983)). Sie sind wesentlich an der Regulation der Bluthomöostase beteiligt, dem Gleichgewicht zwischen Blutgerinnung, Clotbildung und Auflösung. Dieses gut regulierte System kann sowohl durch genetisch bedingte Defekte, wie z.B. der Hämophilie A (defekter Faktor VIII) und Hämophilie B (defekter Faktor IX), als auch durch akute Störungen aus dem Gleichgewicht geraten, wie z.B. beim Herzinfarkt, der Embolie und dem Gehirnschlag.

Es besteht deshalb ein Bedarf an Substanzen, mit denen das System der Blutgerinnung und Fibrinolyse je nach medizinischer Notwendigkeit beeinflußt werden kann. Z.B. wird zur Behandlung der Hämophilie A und B rekombinant hergestellter Faktor VIII bzw. Faktor IX oder seit kurzem auch Faktor VII verwendet. Zur Clotauflösung, z.B. nach Herzinfarkt, findet tPA ("tissue type plasminogen activator") und Streptokinase (bakterielle Protease) Anwendung. Neben komplexen Proteinen werden auch Substanzen, wie z.B. Hirudin (Peptid aus 65 Aminosäuren, Thrombininhibitor), Heparin (Heteroglykan, Thrombininhibition / Cofaktor) und Vitamin-K Antagonisten (Inhibitoren der γ-Carboxylierung; Glu-Reste der GLA-Domäne), zur Hemmung der Blutgerinnung verwendet. Die verfügbaren Substanzen sind jedoch oft noch sehr teuer (Proteinfaktoren) und hinsichtlich ihrer medizinischen Anwendung nicht ideal (Nebenwirkungen), so daß ein Bedarf an Medikamenten besteht, mit dem die Blutgerinnung und Clotauflösung spezifischer moduliert werden kann.

Die Suche nach neuen Modulatoren (Aktivatoren, Inhibitoren) der Blutgerinnung, Fibrinolyse und Homöostase kann z.B. durch Screening von Substanzbibliotheken und anschließende Verbesserung einer identifizierten Leitstruktur durch "drug modelling" erfolgen. Dazu ist es notwendig, daß das (die) Schlüsselprotein(e) [Target(s)] in ausreichender Menge und Qualität für ein Screening und für Kristallisationsuntersuchungen (z.B. Verbesserung der Leitstruktur durch gezielte Vorhersage von Veränderungen anhand der 3D-Struktur aus Proteinkomponente und Leitstruktur) zur Verfügung stehen.

Attraktive Targets innerhalb der Homöostase stellen z.B. die aktivierten Serinproteasen Thrombin, FVIIa, FIXa, FXa, FXIa, FXIIa, Kallikrein (Blutgerinnung), tPA, Urokinase, Plasmin (Fibrinolyse) und aktiviertes Protein C (regulatorischer Antikoagulant) und deren inaktive Vorstufen (Zymogene) dar.

Die Isolierung der inaktiven Serinproteasen (Zymogene) aus Blutplasma und die anschließende Aktivierung durch Proteolyse ist schwierig, aufwendig, teuer und liefert oft nicht die z.B. für Kristallisationsexperimente gewünschte Menge und Qualität. Z.B. beträgt die Plasmakonzentration für die inaktiven Proteasezymogene FX, FIX und FVII nur 10, 5, beziehungsweise 0,5 mg/l (Furie, B. und Furie, B.C., Cell 53 (1988) 505-518). Zudem sind die aus dem Plasma isolierten und in vitro aktivierten Proteasepräparationen oft sehr heterogen und instabil. Außerdem erschweren nicht einheitliche posttranslationale Modifizierungen (z.B. Kohlenhydratgruppen) Kristallisationsexperimente.

Blutplasmaproteasen sind komplexe Glycoproteine, die zur Familie der Serinproteasen gehören. Sie werden in der Leber als inaktive Proenzyme (Zymogene) synthetisiert, ins Blut sezerniert und bei Bedarf durch spezifische Proteolyse, d.h. durch Spaltung von ein oder zwei Peptidbindungen, aktiviert. Sie sind strukturell hinsichtlich der Proteindomänenanordnung und Zusammensetzung sehr ähnlich (Furie, B. und Furie, B.C., Cell 53 (1988) 505-518).

Die Proteasen der Faktor IX Familie (Faktor VII, IX, X, und Protein C) bestehen gemäß Furie, B. und Furie, B.C. aus
― einem Propeptid,
― einer GLA-Domäne,
― einer "aromatic amino acid stack" Domäne,
― zwei EGF-Domänen (EGF1 und EGF2),
― einer Zymogen Aktivierungsdomäne (Aktivierungspeptid, AP) und
― einer katalytischen Proteasedomäne (CD).

Zudem werden die Blutplasmaproteasen während der Sekretion posttranslational modifiziert:
― 11 - 12 Disulfidbrücken
― N- und/oder O-Glycosylierung (GLA-Domäne und Aktivierungspeptid)
   - Bharadwaj, D. et al., J. Biol. Chem. 270 (1995) 6537-6542
   - Medved, L.V. et al., J. Biol. Chem. 270 (1995) 13652-13659
― Abspaltung des Propeptides
― γ-Carboxylierung von Glu-Resten (GLA-Domäne)
― β-Hydroxylierung eines Asp-Restes (EGF-Domänen)
― Spaltung der Zymogenregion (teilweise).

Nach Aktivierung der Zymogene (zymogene Form des Proteins) durch spezifische Spaltung von ein oder zwei Peptidbindungen (Aktivierungspeptid) bestehen die enzymatisch aktiven Proteasen aus zwei Ketten, die entspechend ihrem Molekulargewicht mit schwerer und leichter Kette bezeichnet werden. Die beiden Ketten werden in der Faktor IX Proteasefamilie durch eine intermolekulare Disulfidbrücke zwischen der EGF2-Domäne und der Proteasedomäne zusammengehalten. Die Zymogen-Enzym Transformation (Aktivierung) führt zu Konformationsänderungen innerhalb der Proteasedomäne. Dadurch kann sich eine für die Proteaseaktivität notwendige essentielle Salzbrücke zwischen der N-terminalen Aminosäure der Proteasedomäne und einem Asp-Rest innerhalb der Proteasedomäne ausbilden. Für diese Untergruppe von Serinproteasen ist der N-terminale Bereich sehr kritisch und darf nicht modifiziert werden. Nur dann kann sich die für Serinproteasen typische "active site" mit der katalytischen Triade aus Ser, Asp und His ausbilden (Blow, D.M.: Acc. Chem. Res. 9 (1976) 145-152; Polgar, L.: In: Mechanisms of protease action. Boca Raton, Florida, CRC Press, chapter 3 (1989).

Blutplasmaproteasen können klassisch durch Isolierung der inaktiven Zymogene aus dem Blut und anschließende Aktivierung oder rekombinant durch Expresssion der entsprechenden cDNA in einer geeigneten Säugetier-Zellinie oder Hefe hergestellt werden.

### Herstellung von Blutplasmaproteasen durch Expression / Sekretion der Zymogene bzw. aktiven Proteasen mittels eukaryontischer Wirts-/ Vektorsysteme:

**FVII:** Hagen, F.S. et al., EPS 0200421; Pedersen, A.H. et al., Biochem. 28 (1989) 9391-9336; **FIX:** Lin, S.-W. et al., J. Biol. Chem. 265 (1990) 144-150; **FX:** Wolf, D.L. et al., J. Biol. Chem. 266 (1991) 13726-13730; **Protein C:** Bang, N.U. et al., EPS 0191606.

In der Regel werden Wirtszellen verwendet, die in der Lage sind, die Blutplasmaproteasen während des Sekretionsprozeßes entsprechend dem nativen Enzym posttranslational zu modifizieren. Die Zymogen-Enzym Transformation erfolgt dann nachträglich während der "down stream" Prozessierung, wie z.B. im Falle von Prothrombin oder Faktor X mit einem Aktivator aus Schlangengift (Sheehan, J.P. et al., J. Biol. Chem. 268 (1993) 3639-3645; Fujikawa, K. et al., Biochem. 11 (1972) 4892-4898).

Zwecks Zymogen-Enzym Aktivierung in vivo (bereits während der Sekretion) wurden die natürlichen Zymogenschnittstellen bzw. das gesamte Aktivierungspeptid gegen Proteaseschnittstellen ausgetauscht (mehrere benachbarte basische Aminosäuren), die von im Sekretionsweg der Wirtszelle natürlich vorkommenden spezifisch spaltenden Proteasen wie z.B. Kex2 (Hefe) oder PACE (Säugetier Zellinien) gespalten werden können (**FX:** Wolf, D.L. et al., J. Biol. Chem. 266 (1991) 13726-13730; **Prothrombin:** Holly, R.D. und Fester, D.C., WO 93/13208).

Auch die Herstellung von Proteasevarianten (**FX:** Rezaie, A.R. et al., J. Biol. Chem. 268 (1993) 8176-8180; **FIX:** Zhong, D.G. et al., Proc. Natl. Acad. Sci. USA 91 (1994) 3574-3578), Mutanten (**FX:** Rezaie, A.R. et al., J. Biol. Chem. 269 (1994) 21495-21499; Thrombin: Yee, J. et al., J. Biol. Chem. 269 (1994) 17965-17970; **FVII:** Nicolaisen, E.M. et al., WO 88/10295) und Chimären z.B. aus **FIX** und **FX** (Lin, S.-W. et al., J. Biol. Chem. 265 (1990) 144-150; Hertzberg, M.S. et al., J. Biol. Chem. 267 (1992) 14759-14766) mittels eukaryontischer Wirts-/ Vektorsysteme ist bekannt.

### Nachteile der Expression in eukaryontischen Säugetier Zellinien:

― aufwendig
― limitierend hinsichtlich der Expressionsleistung
― teuer
― posttranslationale Modifikationen

### Herstellung von Blutplasmaproteasen durch Expression in Prokaryonten und anschließende Naturierung des Expressionsprodukts:

Thogersen, H.C. et al. (WO 94/18227) beschreiben die Naturiering von FX Varianten mittels eines zyklischen Naturierungsprozesses, bei dem das inaktive FX Protein mittels eines Metallchelatkomplexes ("poly(His)-affinity handle") in einer Chromatographiesäule fixiert ist.

Dazu wurde ein Fusionsprotein verwendet bestehend aus einer verkürzten FX Variante (EGF1, EGF2 und Proteasedomäne), einer zusätzlichen FXa Proteaseerkennungssequenz und einer aus 6 Histidin-Resten bestehenden Fixierungshilfe am C-Terminus der katalytischen Domäne.

### Nachteile:

― Ein Fusionsprotein aus Protease und Poly-His Fixierungshilfe muß konstruiert werden.
― Der Naturierungsprozeß ist sehr aufwendig.
   - Viele Naturierungszyklen sind notwendig.
   - komplexe Apparatur
   - Die Ausbeute beträgt nur 10%.
― Die Fixierungshilfe muß ggf. nach der Naturierung entfernt werden.
― Durch Autokatalyse wird nur der Poly-His Schwanz, nicht aber die zusätzlich eingeführte FXa Spaltstelle entfernt.

DiBella, E.E. et al. (J. Biol. Chem. 270 (1995) 163-169) beschreiben die Naturierung einer verkürzten Thrombinvariante (Prethrombin-2) aus A-Kette (49 Aminosäuren) und B-Kette (259 Aminosäuren).

Eine analoge Faktor Xa Variante bestehend aus Aktivierungspeptid und Proteasedomäne (siehe Beispiel 4) läßt sich jedoch nicht naturieren. Neben der Zymogenregion (Aktivierungspeptid aus ca. 50 Aminosäuren) ist zur FXa Naturierung die EGF2-Domäne erforderlich. Das gilt für alle Mitglieder der FIX Proteinfamilie (FVII, FIX, FX und Protein C).

Thrombin gehört nicht zur FIX Genfamilie und besitzt anstelle von zwei EGF-Domänen zwei Kringel- Domänen.

Überraschenderweise wurde gefunden, daß enzymatisch aktive Proteine mit Serinproteaseaktivität durch Expression einer entsprechenden DNA in Prokaryonten, Naturierung des Expressionsprodukts und enzymatische Spaltung herstellbar sind, wenn sie zusammengesetzt sind aus Serinproteasedomane (katalytische Domäne), N-terminal verbunden mit einer Zymogenaktivierungsdomäne und einer EGF-Domäne (EGF1 und/oder EGF2).

Die erfindungsgemäßen aktiven und verkürzten Serinproteasen der Faktor IX Familie sind hinsichtlich ihrer Spezifität unverändert (identisch) und können demzufolge sowohl in Aktivitätstests als auch im Screening nach neuen Modulatoren (Aktivatoren, Inhibitoren) verwendet werden.

Es war nicht möglich, durch Expression einer nur für die katalytische Domäne codierenden DNA und Naturierung des inaktiven Expressionsprodukts eine enzymatisch aktive Proteasedomäne herzustellen.

Auch durch N-terminale Proteasedomäne-Fusionsproteine mit selektiver Proteaseschnittstelle (z.B. Enterokinase-Spaltstelle) konnten die gewünschten enzymatisch aktiven Proteasedomänen von z.B. FIXa und FXa nicht hergestellt werden. Die Expressionsprodukte waren gemäß dem Stand der Technik nicht naturierbar.

Gegenstand der Erfindung ist ein nicht glycosyliertes, enzymatisch aktives Protein mit Serinproteaseaktivität und dessen zymogene Vorläufer-Form bestehend aus den folgenden Domänen einer Protease aus der Faktor IX Familie:
a) der katalytischen Domäne, N-terminal verbunden mit
b) einer Zymogenaktivierungsdomäne (Aktivierungspeptid), N-terminal verbunden mit
c) einer EGF2-Domäne (vorzugsweise EGF2 oder EGF1 und EGF2).

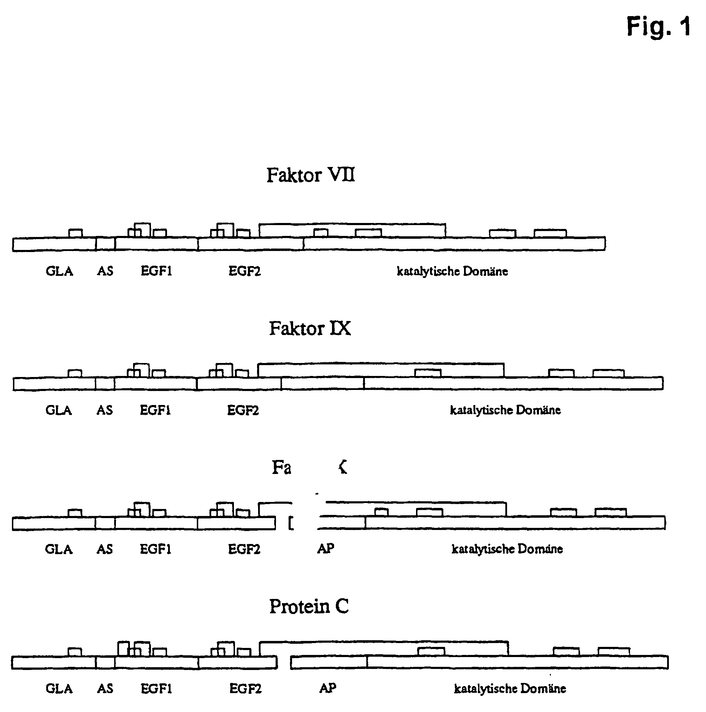

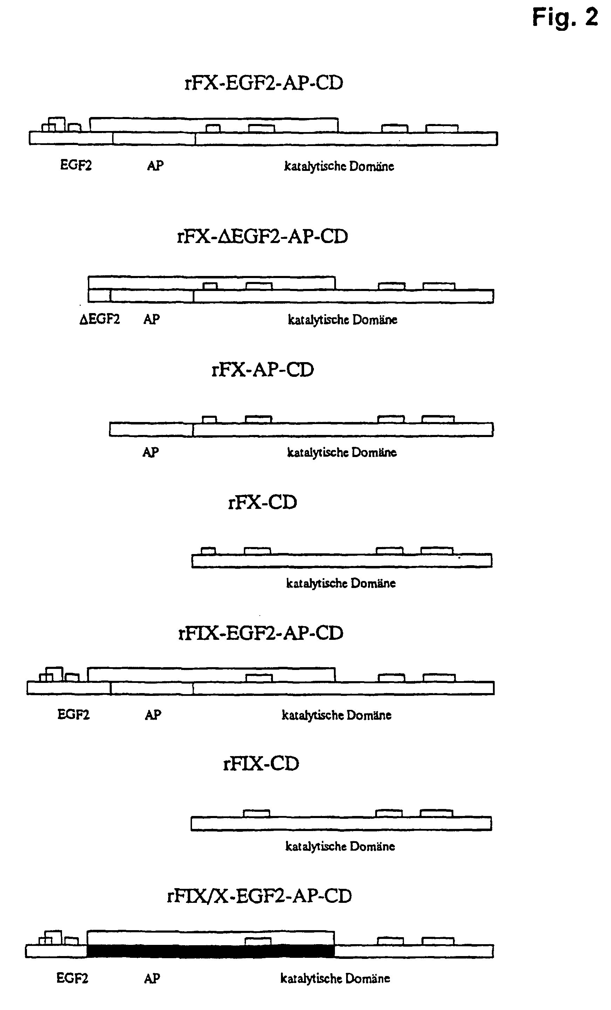

Vorzugsweise besteht die Zymogenaktivierungsdomäne aus einem Oligopeptid mit bis zu 50 Aminosäuren. Nach Spaltung der erfindungsgemäßen zymogenen (inaktiven) einkettigen Form in der Zymogenaktivierungsdomäne entsteht eine zweikettige aktive Protease. In der zweikettigen Form sind die beiden Ketten durch eine intermolekulare Disulfidbrücke (interchain) verbunden (Fig. 1 und Fig. 2).

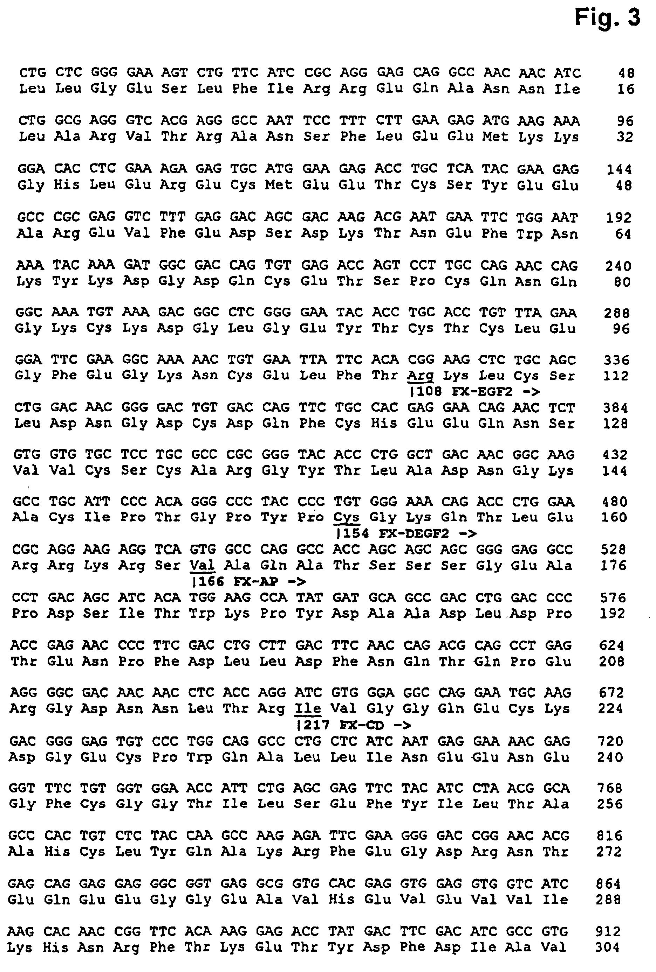

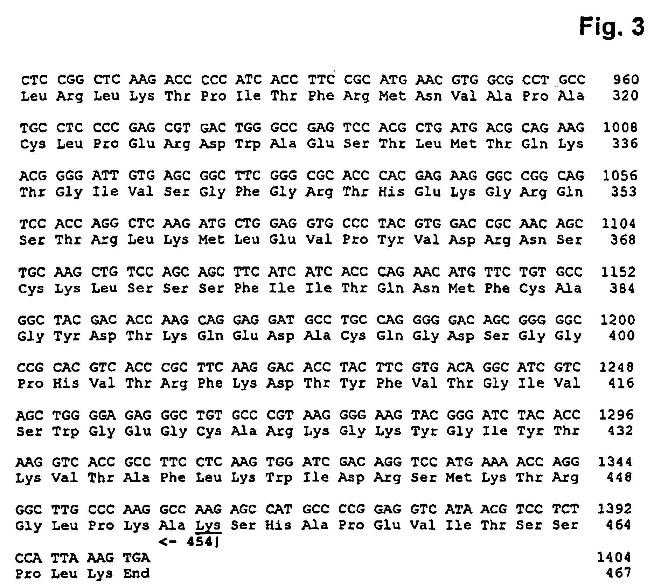

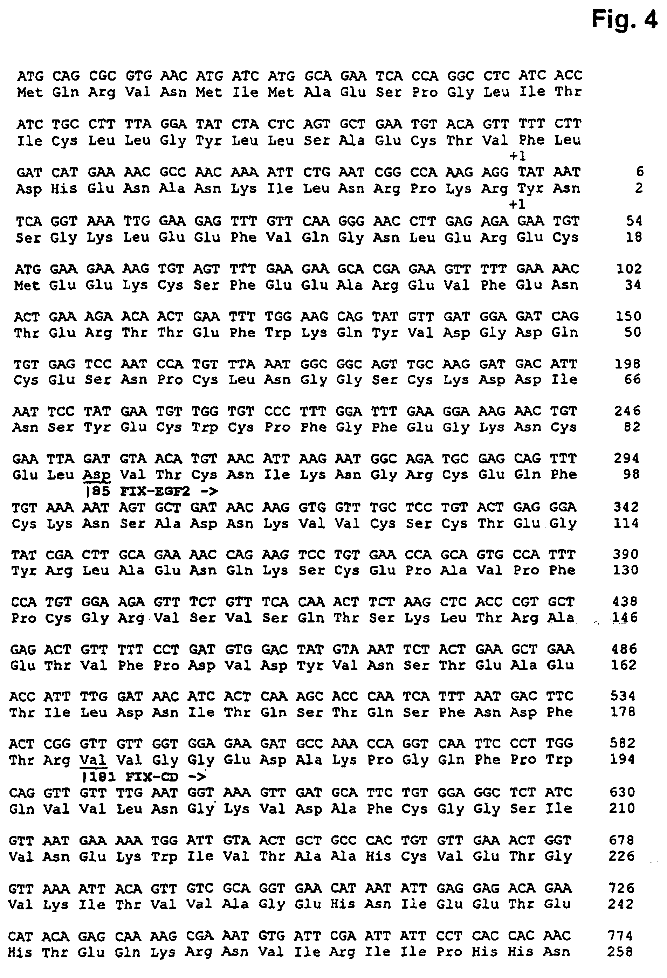

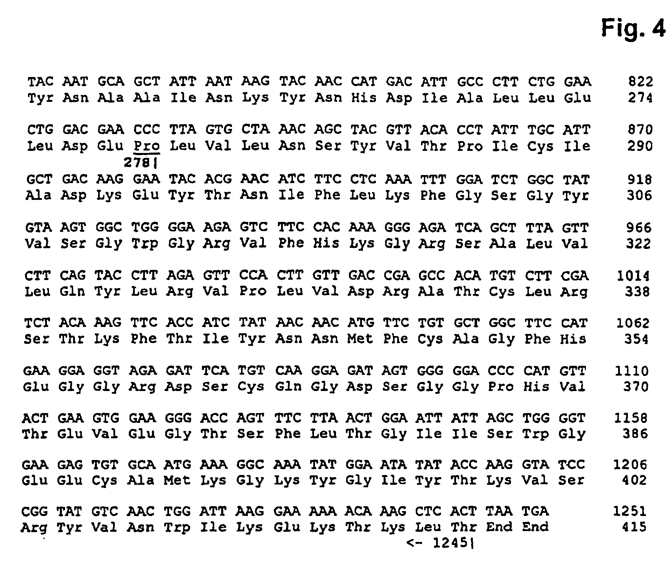

Vorzugsweise bestehen die erfindungsgemäßen Proteine aus der EGF2-Domäne der Zymogenaktivierungsdomäne und der katalytischen Domäne von Faktor X und/oder Faktor IX. Bevorzugt ist ebenfalls ein Protein, welches aus der EGF2-Domäne und der katalytischen Domäne von Faktor X sowie dem Aktivierungspeptid von Faktor IX besteht. Besonders bevorzugt ist ein Protein, welches aus dem N-terminalen Teil der Faktor X EGF2-Domäne (Aminosäureposition 108-154, Fig. 3), dem C-terminalen Teil der Faktor IX EGF2-Domäne, dem Faktor IX Aktivierungspeptid und der Faktor IX N-terminalen Halbseite (Aminosäureposition 133-289, Fig. 4) und der Faktor X C-terminalen Halbseite (Aminosäureposition 322-454, Fig. 3) zusammengesetzt ist.

Die erfindungsgemäßen Zymogene und aktiven Proteasen der Faktor IX Familie können anstelle der natürlichen Zymogene und Proteasen verwendet werden. Zweckmäßige Verwendungsmöglichkeiten sind beispielsweise in der Biotechnologie der Einsatz als Restriktionsprotease (vorzugsweise Faktor Xa), in der Diagnostik als Bestandteil einer enzymatischen Bestimmungsmethode, insbesondere zur indirekten Bestimmung von Blutgerinnungsproteaseaktivitäten (vorzugsweise Faktor IXa-Bestimmung). Eine weitere Verwendungsmöglichkeit ergibt sich als Target in Screening-Assays zur Suche nach Modulatoren (Aktivatoren, Inhibitoren) der Blutgerinnung, Fibrinolyse oder Homöostase. Schließlich stehen mit den erfindungsgemäßen Proteinen kristallisierbare Serinproteasen zur Verfügung, die vorteilhaft für Kristallisationsuntersuchungen (vorzugsweise Cokristallisation mit Aktivatoren und Inhibitoren) eingesetzt werden können.

Besonders bevorzugt werden die erfindungsgemäßen aktiven Proteasen der Faktor IX Familie (Faktor IXa, Faktor Xa, Faktor VIIa und Protein C) zur Identifikation von Inhibitoren verwendet. Ganz besonders bevorzugt ist hierbei die direkte Bestimmung von Faktor IXa und die Identifikation von Faktor IXa Hemmstoffen. Weiterhin können die erfindungsgemäßen Zymogene als Einsatzstoffe in einem diagnostischen Test verwendet werden. Dabei wird das erfindungsgemäße Zymogen (z.B. Faktor X) durch die zu bestimmende Protease (z.B. Faktor IXa) aktiviert. Das aktivierte Zymogen (z.B. Faktor Xa) spaltet dann ein chromogenes Peptidsubstrat (z.B. Chromozym X) und generiert ein Meßsignal (z.B. p-Nitroanilin). Die hierdurch entstandene Farbänderung ist ein Maß für die Konzentration von Faktor IXa in der Probe und ist proportional zur zu bestimmenden Proteaseaktivität.

Vorzugsweise ist zwischen der Zymogenaktivierungsdomäne und der EGF-Domäne (oder den EGF-Domänen) ein Spacer mit bis zu 50 Aminosäuren eingefügt. Bei der Spaltung der erfindungsgemäßen zymogenen einkettigen Form in der Zymogenaktivierungsdomäne wird ein aktives Protein in einer zweikettigen Form erhalten. In der zweikettigen Form sind beide Ketten durch eine intermolekulare Disulfidbrücke verbunden (Fig. 1 und Fig. 2).

Vorzugsweise bestehen die erfindungsgemäßen Proteine aus der EGF2-Domäne, dem Aktivierungspeptid und der katalytischen Domäne von Faktor X und/oder Faktor IX. Bevorzugt ist ebenfalls ein Protein, welches aus der EGF2-Domäne und der katalytischen Domäne von Faktor X sowie der Aktivierungsdomäne von Faktor IX besteht.

### Methoden

### Rekombinante DNA-Technik

Zur Manipulation von DNA wurden Standardmethoden benutzt, wie sie bei Sambrook, J. et al. (1989) In: Molecular cloning: A laboratory manual. Cold Spring Harbor Laboratory Press, Cold Spring Harbor, New York, beschrieben sind. Die verwendeten molekularbiologischen Reagenzien wurden nach den Angaben des Herstellers eingesetzt.

### Proteinbestimmung

Die Proteinkonzentration der Proteasevarianten wurde durch Bestimmung der optischen Dichte (OD) bei 280 nm unter Verwendung des anhand der Aminosäuresequenz errechneten molaren Extinktionskoeffizienten ermittelt.

### Expressionsvektor

Der Vektor zur Expression der Blutgerinnungsproteasevarianten basiert auf dem Expressionsvektor pSAM-CORE für core-Streptavidin. Die Herstellung und Beschreibung des Plasmids pSAM-CORE ist in der WO 93/09144 von Kopetzki, E. et al. beschrieben.

Das core-Streptavidin Gen wurde durch das gewünschte Proteasevariantengen im pSAM-CORE Vektor ersetzt.

Die folgenden Beispiele, Publikationen, das Sequenzprotokoll und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung der Figuren

- Fig. 1: ist eine schematische Darstellung der Blutplasmaproteasen der FIX Proteasefamilie.
- Fig. 2: ist eine schematische Darstellung der konstruierten verkürzten FIX, FX und FIX/X chimären Blutplasmaproteasen.
(Bei rFIX/X-EGF2-AP-CD ist der Faktor X-Teil weiß und der Faktor IX-Teil schwarz.)
Abkürzungen: AP = Aktivierungspeptid; AS = "aromatic amino acid stack" Domäne; CD = katalytische Domäne; EGF1 = epidermale-Wachstumsfaktor-ähnliche Domäne 1; EGF2 = epidermale-Wachstumsfaktor-ähnliche Domäne 2; GLA = γ-carboxyglutaminsäurereiche Domäne.
- Fig. 3: zeigt die für FX bei Kaul, R.K. et al. (Gene 41 (1986) 311-314) angegebene Nukleotid- und Aminosäuresequenz. (Die Nukleotidsequenz ist in SEQ ID NO:15 gezeigt.)
- Fig. 4: zeigt die für FIX bei McGraw, R.A. et al. (Proc. Natl. Acad. Sci. USA 82 (1985) 2847-2851) angegebene Nukleotid- und Aminosäuresequenz. (Die Nukleotidsequenz ist in SEQ ID NO:16 gezeigt.)

### Beispiel 1

### Klonierung der katalytischen Domäne des FX Proteasegens

### (Plasmid: pFX-CD)

Die FX cDNA von Bp-Position 649-1362, kodierend für die FX Proteasedomäne von Aminosäureposition 217-454 (cDNA Sequenz- und Aminosäuresequenz-Numerierung entsprechend der Publikation von Kaul, R.K. et al. (Gene 41 (1986) 311-314; Fig. 3) wurde in einer "Polymerase Chain Reaktion" (PCR) gemäß der Methode von Mullis, K.B. und Faloona, F.A. (Methods Enzymol. 155, (1987) 355-350) unter Verwendung der PCR Primer N1 (SEQ ID NO: 1) und N2 (SEQ ID NO: 2) und einer kommerziell erhältlichen humanen Leber cDNA Genbank (Vektor: Lamba ZAP® II) der Firma Stratagene (La Jolla, CA, U.S.A.) als Template DNA amplifiziert. Mittels der PCR Primer wurde am 5'-Ende der kodierenden Region eine singuläre BspHI Schnittstelle und ein ATG-Startkodon und am 3'-Ende der kodierenden Region eine singuläre HindIII Schnittstelle eingeführt.

Das ca. 740 Bp lange PCR Produkt wurde mit den Restriktionsendonukleasen BspHI und HindIII verdaut und das ca. 725 Bp lange BspHI/HindIII-FX Fragment nach Reinigung durch Agarosegelelektrophorese in das ca. 2,55 kBp lange NcoI/HindIII-pSAM-CORE Vektorfragment ligiert. Das gewünschte Plasmid pFX-CD wurde durch Restriktionskartierung identifiziert und die durch PCR isolierte FX cDNA Sequenz durch DNA Sequenzierung überprüft.

### Beispiel 2

### Konstruktion des FX Proteasegens mit N-terminalem (His)₄-Schwanz, Enterokinasespaltstelle und katalytischer Domäne (Plasmid: pFX-EK-CD)

Der Leserahmen des klonierten FX-CD Gens (siehe Beispiel 1) wurde am 5'-Ende mit einer Nukleotidsequenz verbunden, die für die Aminosäuresequenz MHHHHDDDDK (SEQ ID NO:17) kodiert und das ATG-Startkodon, eine Poly-His-Sequenz und eine Enterokinasespaltstelle enthält. Zur Konstruktion dieses FX-EK-CD Variantengens wurde die am 5'-Ende des FX-CD Gens lokalisierte singuläre BsmI Schnittstelle und die stromaufwärts im Promotor benachbarte singuläre EcoRI Schnittstelle verwendet.

Dazu wurde das Plasmid pFX-CD mit den Restriktionsendonukleasen EcoRI und BsmI verdaut und das ca. 3,25 kBp lange EcoRI/BsmI-pFX-CD Vektorfragment nach Isolierung mittels Agarosegelelektrophorese mit dem FX-EK-CD DNA-Adaptor ligiert. Der FX-EK-CD Adaptor wurde durch Hybridisierung aus den komplementären Oligonukleotiden N3 (SEQ ID NO: 3) und N4 (SEQ ID NO: 4) (Reaktionspuffer: 12,5 mmol/l Tris-HCl, pH 7,0 und 12,5 mmol/l MgCl₂ ; N Konzentration: jeweils 1 pmol / 60 µl) hergestellt.

### FX-EK-CD Adaptor:

### Beispiel 3

### Klonierung des FX Proteasegens mit EGF2-Domäne, Aktivierungspeptid und katalytischer Domäne (Plasmid: pFX-EGF2-AP-CD)

Die FX cDNA von Bp-Position 322-1362, kodierend für die EGF2-Domäne, das Aktivierungspeptid und die katalytische Proteasedomäne von Aminosäureposition 108-454 (cDNA Sequenz- und Aminosäuresequenz-Numerierung entsprechend Fig. 3) wurde mittels PCR unter Verwendung der PCR Primer N5 (SEQ ID NO: 5) und N2 (SEQ ID NO: 2) und einer kommerziell erhältlichen humanen Leber cDNA Genbank (Vektor: Lamba ZAP®II) der Firma Stratagene (La Jolla, CA, U.S.A.) als Template DNA amplifiziert. Mittels der PCR Primer wurde am 5'-Ende der kodierenden Region ein ATG-Startkodon und eine singuläre EcoRI Schnittstelle und am 3'-Ende der kodierenden Region eine singuläre HindIII Schnittstelle eingeführt.

Das ca. 1,09 kBp lange PCR Produkt wurde mit den Restriktionsendonukleasen EcoRI und BstEII verdaut und das ca. 1,02 kBp lange EcoRI/BstEII-FX Fragment nach Reinigung durch Agarosegelelektrophorese in das ca. 2,58 kBp lange EcoRI/BstEII-pFX-CD Vektorfragment (Beispiel 1) ligiert. Das gewünschte Plasmid pFX-EGF2-AP-CD wurde durch Restriktionskartierung identifiziert und die durch PCR isolierte FX cDNA Sequenz durch DNA Sequenzierung überprüft.

### Beispiel 4

### Konstruktion des FX Proteasegens mit verkürzter EGF2-Domäne, Aktivierungspeptid und katalytischer Domäne (Plasmid: pFX-ΔEGF2-AP-CD)

Die FX cDNA von Bp-Position 460-1362, kodierend für eine verkürzte EGF2-Domäne, das Aktivierungspeptid und die katalytische Proteasedomäne von Aminosäureposition 154-454 (cDNA Sequenz- und Aminosäuresequenz-Numerierung entsprechend Fig. 3) wurde mittels PCR unter Verwendung der PCR Primer N6 (SEQ ID NO: 6) und N2 (SEQ ID NO: 2) und dem Plasmid pFX-EGF2-AP-CD (Beispiel 3) als Template DNA amplifiziert. Bei der PCR wurde mittels des N6 Primers der 5'-Bereich des Strukturgens (Aminosäureposition 2 und 3) an die in E. coli bevorzugt benutzten Kodons angepaßt ("ATG-Umgebung mit optimierter Codonusage", gekennzeichnet durch Kleinschreibung der Basen im N6 Primer), ohne die Proteinsequenz zu verändern.

Das ca. 960 Bp lange PCR Produkt wurde mit den Restriktionsendonukleasen EcoRI und HindIII verdaut und das ca. 950 Bp lange EcoRI/HindIII-FX Fragment nach Reinigung durch Agarosegelelektrophorese in das ca. 2,53 kBp lange EcoRI/HindIII-pSAM-CORE Vektorfragment (Beispiel 1) ligiert. Das gewünschte Plasmid pFX-ΔEGF2-AP-CD wurde durch Restriktionskartierung identifiziert und die durch PCR amplifizierte FX DNA Sequenz durch DNA Sequenzierung überprüft.

### Beispiel 5

### Konstruktion des FX Proteasegens mit Aktivierungspeptid und katalytischer Domäne (Plasmid: pFX-AP-CD)

Die FX cDNA von Bp-Position 496-1362, kodierend für das Aktivierungspeptid und die katalytische Proteasedomäne von Aminosäureposition 166-454 (cDNA Sequenz- und Aminosäuresequenz-Numerierung entsprechend Fig. 3) wurde mittels PCR unter Verwendung der PCR Primer N7 (SEQ ID NO: 7) und N2 (SEQ ID NO: 2) und dem Plasmid pFX-EGF2-AP-CD (Beispiel 3) als Template DNA amplifiziert. Bei der PCR wurde mittels des N7 Primers der 5'-Bereich des Strukturgens (Aminosäureposition 2, 3 und 5) an die in E. coli bevorzugt benutzten Kodons angepaßt ("ATG-Umgebung mit optimierter Codonusage", gekennzeichnet durch Kleinschreibung der Basen im N7 Primer), ohne die Proteinsequenz zu verändern.

Das ca. 890 Bp lange PCR Produkt wurde mit den Restriktionsendonukleasen NcoI und HindIII verdaut und das ca. 880 Bp lange NcoI/HindIII-FX Fragment nach Reinigung durch Agarosegelelektrophorese in das ca. 2,55 kBp lange NcoI/HindIII-pSAM-CORE Vektorfragment (Beispiel 1) ligiert. Das gewünschte Plasmid pFX-AP-CD wurde durch Restriktionskartierung identifiziert und die durch PCR amplifizierte FX DNA Sequenz durch DNA Sequenzierung überprüft.

### Beispiel 6

### Klonierung der katalytischen Domäne des FIX Proteasegens (Plasmid: pFIX-CD)

Die FIX cDNA von Bp-Position 690-1403, kodierend für die FIX Proteasedomäne von Aminosäureposition 181-415 (cDNA Sequenz- und Aminosäuresequenz-Numerierung entsprechend der Publikation von McGraw, R.A. et al. (Proc. Natl. Acad. Sci. USA 82 (1985) 2847-2851; Fig. 4) wurde unter Verwendung der PCR Primer N8 (SEQ ID NO: 8) und N9 (SEQ ID NO: 9) und einer kommerziell erhältlichen humanen Leber cDNA Genbank (Vektor: Lamba ZAP®II) der Firma Stratagene (La Jolla, CA, U.S.A.) als Template DNA amplifiziert. Mittels der PCR Primer wurde am 5'-Ende der kodierenden Region eine singuläre NcoI Schnittstelle und ein ATG-Startkodon und am 3'-Ende der kodierenden Region eine singuläre HindIII Schnittstelle eingeführt.

Das ca. 730 Bp lange PCR Produkt wurde mit den Restriktionsendonukleasen NcoI und HindIII verdaut und das ca. 720 Bp lange NcoI/HindIII-FIX Fragment nach Reinigung durch Agarosegelelektrophorese in das ca. 2,55 kBp lange NcoI/HindIII-pSAM-CORE Vektorfragment ligiert (Beispiel 1). Das gewünschte Plasmid pFIX-CD wurde durch Restriktionskartierung identifiziert und die durch PCR isolierte FIX cDNA Sequenz durch DNA Sequenzierung überprüft.

### Beispiel 7

### Konstruktion des FIX Proteasegens mit EGF2-Domäne, Aktivierungspeptid und katalytischer Domäne (Plasmid: pFIX-EGF2-AP-CD)

Die FIX cDNA von Bp-Position 402-986, kodierend für die EGF2-Domäne, das Aktivierungspeptid und den N-terminalen Bereich der FIXa Proteasedomäne von Aminosäureposition 85-278 (cDNA Sequenz- und Aminosäuresequenz-Numerierung entsprechend Fig. 4) wurde unter Verwendung der PCR Primer N10 (SEQ ID NO: 10) und N11 (SEQ ID NO: 11) und einer kommerziell erhältlichen humanen Leber cDNA Genbank (Vektor: Lamba ZAP® II) der Firma Stratagene (La Jolla, CA, U.S.A.) als Template DNA amplifiziert. Mittels des PCR Primers N10 wurde am 5'-Ende der kodierenden Region ein ATG-Startkodon und eine singuläre NcoI Schnittstelle eingeführt.

Das ca. 590 Bp lange PCR Produkt wurde mit den Restriktionsendonuldeasen NcoI und BsmI verdaut und das ca. 360 Bp lange NcoI/BsmI-FIX-EGF2-AP Fragment nach Reinigung durch Agarosegelelektrophorese in das ca. 3,2 kBp lange NcoI/BsmI-pFIX-CD Vektorfragment (Beispiel 6) ligiert. Das gewünschte Plasmid pFIX-EGF2-AP-CD wurde durch Restriktionskartierung identifiziert und die durch PCR isolierte FIX cDNA Sequenz durch DNA Sequenzierung überprüft.

### Beispiel 8

### Konstruktion eines chimären Proteasegens aus FTX und FX (Plasmid: pFIX/X-EGF2-AP-CD)

Das chimäre FIX/FX Proteasegen wurde aus dem N-terminalen Teil der FX EGF2-Domäne (Bp-Position: 322-462; Aminosäureposition: 108-154, Fig. 3), dem C-terminalen Teil der FIX EGF2, dem FIX Aktivierungspeptid und der FIX N-terminalen Halbseite (Bp-Position: 397-867; Aminosäureposition: 133-289, Fig. 4) und der FX C-terminalen Halbseite (Bp-Position: 964-1362; Aminosäureposition: 322-454; Fig. 3) zusammengesetzt.

Dazu wurde in einer ersten PCR Reaktion die DNA kodierend für den C-terminalen Teil der FIX EGF2, das FIX Aktivierungspeptid und die FIX N-terminale Halbseite von Bp-Position: 397-867 (Aminosäureposition: 133-289; Fig. 4) unter Verwendung der PCR Primer N12 (SEQ ID NO: 12) und N13 (SEQ ID NO: 13) und des Plasmids pFIX-EGF2-AP-CD (Beispiel 7) als Template DNA amplifiziert. Mittels der 5'-überhängenden Nukleotidsequenz des PCR Primers N12 wurde die FX-EGF2- mit der FIX-EGF2 DNA Sequenz verbunden. Sie besteht aus der FX DNA Sequenz von Bp-Position 430-462 (Fig. 3) mit einer singulären StuI Schnittstelle am 5'-Ende. Mit der 5'-überhängenden Nukleotidsequenz des N13 Primers wurde die FIX- mit der FX DNA verbunden. Sie besteht aus der FX DNA Sequenz von Bp-Position: 964-970 (Fig. 3). Durch 2 Bp-Substitutionen (durch Kleinschreibung der Basen im N13 Primer gekennzeichnet) wurde in dieser Sequenz eine singuläre MroI Schnittstelle erzeugt, ohne die Proteinsequenz zu verändern. In einer zweiten PCR Reaktion wurde die FX C-terminale Halbseite von Bp-Position: 964-1362 (Aminosäureposition: 322-454; Fig. 3) unter Verwendung der PCR Primer N14 (SEQ ID NO: 14) und N2 (SEQ ID NO: 2) und des Plasmids pFX-EGF2-AP-CD (Beispiel 3) als Template DNA amplifiziert. Mittels des N14 Primers wurde am 5'-Ende innerhalb der kodierenden FX-CD Region durch 2 Bp-Substitutionen (durch Kleinschreibung der Basen im N14 Primer gekennzeichnet) eine singuläre MroI Schnittstelle eingeführt, ohne die Aminosäuresequenz zu verändern.

Das erste PCR Produkt wurde mit StuI und MroI und das zweite PCR Produkt mit MroI und HindIII verdaut. Danach wurde in einer Dreifragmentligation das ca. 510 Bp lange StuI/MroI-Fragment mit dem ca. 400 Bp langen MroI/HindIII-Fragment und dem ca. 2640 Bp langen StuI/HindIII-pFX-EGF2-AP-CD Vektorfragment (Beispiel 3) ligiert. Das gewünschte Plasmid pFIX/X-EGF2-AP-CD wurde durch Restriktionskartierung identifiziert und die durch PCR amplifizierte FIX/X DNA Sequenz durch DNA Sequenzierung überprüft.

### Beispiel 9

### a) Expression der Proteasegene in E. coli

Zur Expression der Proteasegene wurde der E. coli K12 Stamm UT5600 (Grodberg, J. und Dunn, J.J., J. Bacteriol. 170 (1988) 1245-1253) jeweils mit einem der in den Beispielen 1-8 beschriebenen Expressionsplasmiden pFX-CD, pFX-EK-CD, pFX-EGF2-AP-CD, pFX-ΔEGF2-AP-CD, pFX-AP-CD, pFIX-CD, pFIX-EGF2-AP-CD und pFIX/X-EGF2-AP-CD (Ampicillin-Resistenz) und dem lacI^{q}-Repressorplasmid pUBS520 (Kanamycin-Resistenz, Herstellung und Beschreibung siehe: Brinkmann, U. et al., Gene 85 (1989) 109-114) transformiert.

Die mit den Expressionsplasmiden pFX-CD, pFX-EK-CD, pFX-EGF2-AP-CD, pFX-ΔEGF2-AP-CD, pFX-AP-CD, pFIX-CD, pFIX-EGF2-AP-CD und pFIX/X-EGF2-AP-CD transformierten UT5600/pUBS520/Zellen wurden in Schüttelkultur in DYT-Medium (1% (w/v) Hefeextrakt, 1% (w/v) Bacto Tryptone, Difco, und 0,5% NaCl) mit 50-100 mg/l Ampicillin und 50 mg/l Kanamycin bei 37°C bis zu einer optischen Dichte bei 550 nm (OD₅₅₀) von 0,6-0,9 angezogen und anschließend mit IPTG (1-5 mmol/l Endkonzentration) induziert. Nach einer Induktionsphase von 4 - 8 Stunden (Std.) bei 37°C wurden die Zellen durch Zentrifugation (Sorvall RC-5B Zentrifuge, GS3 Rotor, 6000 UPM, 15 min) geerntet, mit 50 mmol/l Tris-HCl Puffer, pH 7,2 gewaschen und bis zur Weiterverarbeitung bei -20°C gelagert. Die Zellausbeute aus einer 11 Schüttelkultur betrug 4-5 g (Naßgewicht).

### b) Expressionsanalyse

Die Expression der mit den Plasmiden pFX-CD, pFX-EK-CD, pFX-EGF2-AP-CD, pFX-ΔEGF2-AP-CD, pFX-AP-CD, pFIX-CD, pFIX-EGF2-AP-CD und pFIX/X-EGF2-AP-CD transformierten UT5600/pUBS520/Zellen wurde analysiert. Dazu wurden Zellpellets aus jeweils 1 ml abzentrifugiertem Anzuchtmedium in 0,25 ml 10 mmol/l Tris-HCl, pH 7,2 resuspendiert und die Zellen durch Ultraschallbehandlung (2 Pulse a 30 s mit 50% Intensität) mit einem Sonifier® Cell Disruptor B15 der Firma Branson (Heusenstamm, BRD) aufgeschlossen. Die unlöslichen Zellbestandteile wurden sedimentiert (Eppendorf 5415 Zentrifuge, 14000 UPM, 5 min) und der Überstand mit 1/5 Volumen (Vol) 5xSDS-Probenpuffer (1xSDS-Probenpuffer: 50 mmol/l Tris-HCl, pH 6,8, 1% SDS, 1% Mercaptoethanol, 10% Glycerin, 0.001% Bromphenolblau) versetzt. Die unlösliche Zelltrümmerfraktion (Pellet) wurde in 0,3 ml 1xSDS-Probenpuffer mit 6 - 8 M Harnstoff resuspendiert, die Proben 5 min bei 95°C inkubiert und erneut zentrifugiert. Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Die in E. coli synthetisierten Proteasevarianten waren homogen und wurden ausschließlich in der unlöslichen Zelltrümmerfraktion gefunden ("inclusion bodies", IBs). Die Expressionshöhe betrug 10-50% bezogen auf das E. coli Gesamtprotein.

### Beispiel 10

### Zellyse, Solubilisierung und Naturierung der Proteasevarianten

### a) Zellyse und Präparation der "inclusion bodies" (IBs)

Das Zellpellet aus 3 l Schüttelkultur (ca. 15 g Naßgewicht) wurde in 75 ml 50 mmol/l Tris-HCl, pH 7,2, resuspendiert. Die Suspension wurde mit 0,25 mg/ml Lysozym versetzt und 30 min bei 0°C inkubiert. Nach Zugabe von 2 mmol/l MgCl₂ und 10 µg/ml DNase I (Boehringer Mannheim GmbH, Kat.-Nr. 104159) wurden die Zellen mechanisch mittels Hochdruckdispersion in einer French® Press der Firma SLM Amico (Urbana, IL, U.S.A.) aufgeschlossen.

Anschließend wurde die DNA 30 min bei Raumtemperatur (RT) verdaut. Der Ansatz wurde mit 37,5 ml 50 mmol/l Tris-HCl pH 7,2, 60 mmol/l EDTA, 1,5 mol/l NaCl, 6% Triton X-100 versetzt, weitere 30 min bei RT inkubiert und in einer Sorvall RC-5B Zentrifuge (GSA Rotor, 12000 UPM, 15 min) zentrifugiert. Der Überstand wurde verworfen, das Pellet mit 100 ml 50 mmol/l Tris-HCl, pH 7,2, 20 mmol/l EDTA versetzt, 30 min unter Rühren bei 4°C inkubiert und erneut sedimentiert. Der letzte Waschschritt wurde wiederholt. Die gereinigten IBs (1,5-2,0 g Naßgewicht, 25-30% Trockenmasse, 100-150 mg Protease) wurden bis zur Weiterverarbeitung bei -20°C gelagert.

### b) Solubilisierung und Derivatisierung der IBs

Die gereinigten IBs wurden in einer Konzentration von 100 mg IB-Pellet (Naßgewicht)/ml entsprechend 5-10 mg/ml Protein in 6 mol/l Guanidinium-HCl, 100 mmol/l Tris-HCl, 20 mmol/l EDTA, 150 mmol/l GSSG und 15 mmol/l GSH, pH 8,0 unter Rühren bei RT in 1-3 Std. gelöst. Anschließend wurde der pH auf pH 5,0 eingestellt und die unlöslichen Bestandteile durch Zentrifugation (Sorvall RC-5B Zentrifuge, SS34 Rotor, 16000 UPM, 10 min) abgetrennt. Der Überstand wurde gegen 100 Vol 4-6 mol/l Guanidinium-HCl pH 5,0 für 24 Std. bei 4°C dialysiert.

### c) Naturierung

Die Naturierung der in 6 mol/l Guanidinium-HCl solubilisierten und mit GSSG/GSH derivatisierten Proteasevarianten wurde bei 4°C durch wiederholte (z.B. 3-fache) Zugabe von jeweils 0,5 ml IB-Solubilisat/Derivat zu 50 ml 50 mmol/l Tris-HCl, 0,5 mol/l Arginin, 20 mmol/l CaCl₂, 1 mmol/l EDTA und 0,5 mmol/l Cystein, pH 8,5 im Zeitabstand von 24 Std. und anschließende Inkubation für 48 Std. bei 4°C bewirkt. Nach Abschluß der Naturierungsreaktion wurden unlösliche Bestandteile durch Filtration mit einem Filtrationsgerät der Firma Satorius (Göttingen, BRD) bestückt mit Tiefenfilter K 250 der Firma Seitz (Bad Kreuznach, BRD) abgetrennt.

### d) Konzentrierung und Dialyse der Naturierungsansätze

Der klare proteasehaltige Überstand wurde durch Cross-Flow-Filtration in einer Minisette (Membran Typ: Omega 10K) der Firma Filtron (Karlstein, BRD) 10-15-fach konzentriert und zur Entfernung von Guanidinium-HCl und Arginin gegen 100 Vol 20 mmol/l Tris-HCl und 50 mmol/l NaCl, pH 7,2 für 24 Std. bei 4°C dialysiert. Präzipitiertes Protein wurde durch Zentrifugation abgetrennt (Sorvall RC-5B Zentrifuge, SS34 Rotor, 16000 UPM, 20 min) und der klare Überstand mit einer Nalgene® Einmalfiltrationseinheit (Porendurchmesser: 0,2 µm) der Firma Nalge (Rochester, NY, U.S.A.) filtriert.

### e) Bestimmung der Naturierungseffizienz

Die Proteinkonzentration der naturierten konzentrierten und filtrierten Naturierungsansätze wurde durch Messung der optischen Dichte (OD) bei 280 nm unter Verwendung der anhand der Aminosäuresequenzen errechneten molaren Extinktionskoeffizienten für rFX-CD, rFX-EK-CD, rFX-EGF2-AP-CD, rFX-ΔEGF2-AP-CD, rFX-AP-CD, rFIX-CD, rFIX-EGF2-AP-CD und rFIX/X-EGF2-AP-CD ermittelt.

Eine Probe der Naturierungsansätze, bestehend aus nativ gefalteter Protease und falsch disulfidverbrückten Proteaseoligomeren, wurde durch nicht-reduzierende SDS PAGE aufgetrennt (Beispiel 13 b). Die gewünschten löslichen monomeren Proteasezymogene wurden anhand des apparenten Molekulargewichts und der Bandenschärfe identifiziert. Aus dem Vergleich (Verhältnis) der Bandenintensitäten von monomerem Proteasezymogen zu den restlichen Banden ("Proteinschmier") wurde die "Naturierungseffizienz" abgeschätzt.

| **Proteasevariante** | **molarer Extinktionskoeffizient** **[cm**^{**2**} **mol**^{**-1**}**]** | **Molekulargewicht** **[kDa]** | **Naturierungseffizienz** **[%]** |
|---|---|---|---|
| rFX-CD | 33540 | 27.3 | < 0.1 |
| rFX-EK-CD | 33540 | 28.4 | < 0.1 |
| rFX-EGF2-AP-CD | 43490 | 39.3 | 5-10 |
| rFX-ΔEGF2-AP-CD | 40570 | 34.3 | < 0.1 |
| rFX-AP-CD | 40510 | 32.9 | < 0.1 |
| rFIX-CD | 41670 | 26.3 | < 0.1 |
| rFIX-EGF2-AP-CD | 44650 | 36.9 | 15-20 |
| rFIX/X-EGF2-AP-CD | 43370 | 37.5 | 10-15 |

### Ergebnis:

Nur die Proteasevarianten mit EGF2-Domäne, Aktivierungspeptid (AP) und katalytischer Domäne (CD) ließen sich naturieren.

### Beispiel 11

### Reinigung der naturierten inaktiven Proteasevarianten

Die inaktiven Proteasevarianten aus den Naturierungsansätzen können bei Bedarf mit chromatographischen Methoden, die dem Fachmann bekannt sind, weiter gereinigt werden.

### a) Reinigung der Proteasevarianten durch Ionenaustauschchromatographie an Q-Sepharose-ff

Der konzentrierte und gegen 20 mmol/l Tris-HCl und 50 mmol/l NaCl, pH 8,0 dialysierte Naturierungsansatz wurde auf eine mit dem gleichen Puffer äquilibrierte Q-Sepharose-ff Säule (1,5 x 11 cm, V = 20 ml; Beladungskapazität: 10 mg Protein / ml Gel) der Firma Pharmacia Biotech (Freiburg, BRD) aufgetragen (2 Säulenvolumen/Stunde, 2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 50-500 mmol/l NaCl in 20 mmol/l Tris-HCl, pH 8,0 (2 SV/Std.). Die Proteasen wurden bei einer NaCl Konzentration von 100-150 mmol/l eluiert. Die proteasehaltigen Fraktionen wurden durch nicht-reduzierende und reduzierende SDS PAGE identifiziert und der Elutionspeak vereinigt.

### b) Endreinigung der inaktiven Proteasevarianten durch Ionenaustauschchromatographie an Heparinsepharose CL-6B

Die vereinigten proteasehaltigen Fraktionen nach Chromatographie an Q-Sepharose-ff wurden direkt auf eine mit 20 mmol/l Tris-HCl und 200 mmol/l NaCl, pH 8,0 äquilibrierte Heparinsepharose CL-6B Säule (1,5 x 11 cm; V = 20 ml, Beladungskapazität: 1 mg Protein / ml Gel) der Firma Pharmacia Biotech (Freiburg, BRD) aufgetragen (2 SV/Std.). Danach wurde so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 0,2-1,0 mol/l NaCl in 20 mmol/l Tris-HCl, pH 8,0 (2 SV/Std.). Die Proteasen wurden bei einer NaCl Konzentration von 500-600 mmol/l eluiert. Die proteasehaltigen Fraktionen wurden durch nicht-reduzierende und reduzierende SDS PAGE identifiziert, der Elutionspeak vereinigt und gegen 20 mmol/l Tris-HCl, 50-200 mmol/l NaCl, 5 mmol/l CaCl₂, pH 7,8 dialysiert.

### Beispiel 12

### Aktivierung und Reinigung der aktivierten Proteasevarianten

Die naturierten gereinigten inaktiven rFIX- und rFX Proteasevarianten wurden mit gereinigter "Russels viper venom" (RVV-X) Protease aktiviert. Die RVV-X Protease wurde, wie in der Publikation von Esmon, C.T. (Prothrombin activation, doctoral dissertation, Washington University, St. Louis, M0 (1973)) beschrieben, aus dem kommerziell erhältlichen Schlagengift Lyophilisat der Firma Sigma Aldrich Chemie GmbH (Deisenhofen, BRD) durch Gelfiltration gefolgt von Ionenaustausch-Chromatographie an Q-Sepharose-ff gereinigt.

### a) Aktivierung und Reinigung der rFIX-EGF2-AP-CD Proteasevariante mit RVV-X

Die Proteasevariante rFIX-EGF2-AP-CD wurde in einer Konzentration von 0,5 bis 2,0 mg/ml und einem Protease/Substrat-Verhältnis von 1:10 bis 1:20 in 20 mmol/l Tris-HCl, 50 mmol/l NaCl, 10 mmol/l CaCl₂, pH 7,8 bei 25°C verdaut. Der zeitliche Verlauf der enzymatischen FIX Aktivierung wurde durch Aktivitätsbestimmung mit einem chromogenen Substrat (siehe Beispiel 13 a) bis zur Vollständigkeit des Verdaus (Plateau, maximale Aktivierung) verfolgt. Dazu wurden aus dem Reaktionsansatz über einen Zeitraum von bis zu 24 Std. Proben (10 bis 100 µl) im Abstand von 3-4 Std. entnommen und die generierte rFIXa Aktivität bestimmt. Nach Erreichen des Aktivierungsplateaus wurde der RVV-X Verdau durch Negativ-Chromatographie an Q-Sepharose-ff gereinigt.

RVV-X und nicht aktivierte rFIX-EGF2-AP-CD Protease binden unter den vorgegebenen Bedingungen an Q-Sepharose-ff, die aktivierte rFIXa-EGF2-AP-CD Protease jedoch nicht.

Der Verdauansatz wurde auf eine mit 20 mmol/l Tris-HCl, 50 mmol/l NaCl, pH 7,8 äquilibrierte Q-Sepharose-ff-Säule (1,0 x 10 cm, V = 8 ml) der Firma Pharmacia Biotech (Freiburg, BRD) aufgetragen (2 SV/Std.) und die Säule mit Äquilibrierungspuffer unter Fraktionierung entwickelt. Die rFIXa-EGF2-AP-CD proteasehaltigen Fraktionen wurden durch nicht-reduzierende und reduzierende SDS PAGE und Aktivitätsbestimmung identifiziert.

### b) Aktivierung und Reinigung der rFX-EGF2-AP-CD Proteasevariante mit RVV-X

Die Proteasevariante rFX-EGF2-AP-CD wurde in einer Konzentration von 0,5 bis 2,0 mg/ml und einem Protease/Substrat-Verhältnis von 1:100 bis 1:200 in 20 mmol/l Tris-HCl, 50 mmol/l NaCl, 10 mmol/l CaCl₂, pH 7,8 bei 25°C verdaut. Der zeitliche Verlauf der enzymatischen rFX-EGF2-AP-CD Aktivierung wurde durch Aktivitätsbestimmung mit einem chromogenen Substrat (siehe Beispiel 13 a) bis zur Vollständigkeit des Verdaus (Plateau, maximale Aktivierung) verfolgt. Dazu wurden aus dem Reaktionsansatz über einen Zeitraum von bis zu 4 Std. Proben (10 bis 100 µl) im Abstand von 15-30 min entnommen und die generierte FXa Aktivität bestimmt. Nach Erreichen des Aktivierungsplateaus wurde die aktive rFXa-EGF2-AP-CD Protease durch Chromatographie an Benzamidin-Sepharose-CL-6B gereinigt.

Nur die aktivierte rFXa-EGF2-AP-CD Proteasevariante bindet unter den vorgegebenen Bedingungen an Benzamidin-Sepharose-CL-6B.

Der Verdauansatz wurde auf eine mit 20 mmol/l Tris-HCl, 200 mmol/l NaCl, pH 8,0 äquilibrierte Benzamidin-Sepharose-CL-6B Säule (1,0 x 10 cm, V = 8 ml; Beladungskapazität: 2-3 mg Protein / ml Gel) der Firma Pharmacia Biotech (Freiburg, BRD) aufgetragen (2 SV/Std.) und so lange mit dem Äquilibrierungspuffer gewaschen, bis die Absorption des Eluats bei 280 nm den Leerwert des Puffers erreichte. Die Elution des gebundenen Materials erfolgte durch 10 mmol/l Benzamidin in 20 mmol/l Tris-HCl, 200 mmol/ NaCl, pH 8,0 (2 SV/Std.). Die rFXa-EGF2-AP-CD proteasehaltigen Fraktionen wurden durch nicht-reduzierende und reduzierende SDS PAGE und Aktivitätsbestimmung identifiziert.

### c) Aktivierung mit RVV-X und Reinigung der chimären rFIX/X-EGF2-AP-CD Proteasevariante

Die Proteasevariante rFIX/X-EGF2-AP-CD wurde in einer Konzentration von 0,5 bis 2,0 mg/ml und einem Protease/Substrat-Verhältnis von 1:10 bis 1:20 in 20 mmol/l Tris-HCl, 50 mmol/l NaCl, 10 mmol/l CaCl₂, pH 7,8 bei 25°C verdaut. Der zeitliche Verlauf der enzymatischen rFIX/X-EGF2-AP-CD Aktivierung wurde durch Aktivitätsbestimmung mit einem chromogenen Substrat (siehe Beispiel 13 a) bis zur Vollständigkeit des Verdaus (Plateau, maximale Aktivierung) verfolgt. Dazu wurden aus dem Reaktionsansatz über einen Zeitraum von bis zu 24 Std. Proben (10 bis 100 µl) im Abstand von 3-4 Std. entnommen und die generierte rFIX/Xa-EGF2-AP-CD Aktivität bestimmt. Nach Erreichen des Aktivierungsplateaus wurde der RVV-X Verdau durch Negativ-Chromatographie an Q-Sepharose-ff gereinigt.

RVV-X und nicht aktivierte rFIX/X-EGF2-AP-CD Protease binden unter den vorgegebenen Bedingungen an Q-Sepharose-ff, die aktivierte rFIX/Xa-EGF2-AP-CD Proteasevariante jedoch nicht.

Der Verdauansatz wurde auf eine mit 20 mmol/l Tris-HCl, 50 mmol/l NaCl, pH 7,8 äquilibrierte Q-Sepharose-ff Säule (1,0 x 10 cm, V = 8 ml) der Firma Pharmacia Biotech (Freiburg, BRD) aufgetragen (3 SV/Std.) und die Säule mit Äquilibrierungspuffer unter Fraktionierung entwickelt. Die rFIX/Xa-EGF2-AP-CD proteasehaltigen Fraktionen wurden durch nicht-reduzierende und reduzierende SDS PAGE und Aktivitätsbestimmung identifiziert.

### Beispiel 13

### Charakterisierung der gereinigten Proteasevarianten

### a) Aktivitätstest

Die Aktivität der naturierten aktivierten rFIXa-EGF2-AP-CD, rFXa-EGF2-AP-CD und rFIXa/Xa-EGF2-AP-CD Proteasevarianten wurde mit dem chromogenen Substrat Chromozym X (Boehringer Mannheim GmbH, Mannheim, BRD, Kat.-Nr. 789763) bestimmt. 10-100 µl Probe wurden mit 190-100 µl 50 mmol/l Tris-HCl, 150 mmol/l NaCl, 5 mmol/l CaCl₂, 0,1% Polyethylenglycol 8K (PEG 8000), pH 8,0, auf 200 µl aufgefüllt, mit 20 µl Chromozym X (0,5-40 mmol/l) versetzt und in einem ELISA-Reader bei einer Wellenlänge von 405 nm und RT gegen einen Reagenzienleerwert vermessen. Die Aktivität und die kinetischen Konstanten wurden aus der linearen Anfangssteigung gemäß der Michaelis-Menten-Gleichung bestimmt.

### b) SDS-PAGE

Sowohl die Oligomeren- und Aggregatbildung durch intermolekulare Disulfidbrückenbildung als auch die Homogenität und Reinheit der naturierten aktivierten und gereinigten Proteasevarianten wurde durch nicht-reduzierende (minus Mercaptoethanol) und reduzierende (plus Mercaptoethanol) SDS PAGE (Laemmli, U.K., Nature 227 (1970) 680-685) untersucht.

### Beispiel 14

### FX Aktivatortest

Das rekombinant hergestellte hochreine inaktive rFX-EGF2-AP-CD Zymogen (frei von jeglicher störender Nebenaktivität) eignet sich hervorragend z.B. zur Bestimmung geringer FIXa Konzentrationen in wäßrigen Lösungen, vorzugsweise in Körperflüssigkeiten wie Blut oder Plasma. FIXa aktiviert durch Spaltung das inaktive rFX-EGF2-AP-CD Zymogen. Die Zymogenaktivierung wird durch eine gekoppelte Indikatorreaktion mit einem chromogenen FXa Peptidsubstrat wie z.B. Chromozym X gemessen. Die zu bestimmende FIXa Aktivität wird durch das Verstärkersystem der Zymogenaktivierung amplifiziert. Solch eine FIXa Test ist z.B. bei Van Dam-Mieras, M.C.E. et al., In: Bergmeyer, H.U. (ed.), Methods of Enzymatic Analysis, Vol. V, page 365-394, 3rd ed., Academic Press, New York (1983) beschrieben.

### Testprinzip:

| | |
|---|---|
| Meßsignal | pNA (p-Nitroanilin) |
| FXa Substrat | MOC-D-NleGlyArg-pNA (Chromozym X) |

| | |
|---|---|
| Testansatz | 200 µl Puffer |
| | + 20 µl rFX-EGF2-AP-CD (0,13 mg/ml; 4 µmol/l) |
| | + 25 µl Substrat (Chromozym X, 8 mmol/l) |
| | + 20 µl FIXa Probe |

| | |
|---|---|
| Puffer | 50 mmol/l Tris HCl, pH 8,0; 150 mmol/l NaCl; 5 mmol/l CaCl₂; 0,1 % PEG 8000 |

Der Testansatz wurde in einer Mikrotiterplatte bei RT inkubiert und die Extinktion bei 405 nm gegen einen Reagenzienteerwert in Abhängigkeit von der Zeit bestimmt. Die direkte Umsetzung von Chromozym X durch FIXa ist unter den vorgegebenen Testbedingungen vernachlässigbar.

Die Faktor IXa katalysierte Aktivierung des Zymogens rFX-EGF2-AP-CD wird mit dem chromogenen Peptidsubstrat Chromozym X gemessen. Die Entstehung von p-Nitroanilin (Meßsignal) ist ein Maß (proportional) für die vorhandene (zu bestimmende) Faktor IX Aktivität.

### Beispiel 15

### Kristallisation von rFXa-EGF2-AP-CD

Die aktivierte gereinigte rekombinant hergestellte rFXa-EGF2-AP-CD Protease wurde gegen 2 x 100 Vol 5 mmol/l HEPES Puffer, pH 6,5 bei 4°C für 6 Std. dialysiert und anschließend in einem Centrikon® 10 Mikrokonzentrator der Firma Amicon (Witten, BRD) auf eine Konzentration von 10 mg/ml eingeengt. Die Kristallisation erfolgte durch Dampfdiffusion in einem sitzenden Tropfen. 4 µl konzentrierte rFXa-EGF2-AP-CD Protease (in äquimolarer Konzentration mit dem Inhibitor H-Glu-Gly-Arg-Chlormethylketon (Bachem Biochemica GmbH, Heidelberg, BRD) wurden mit 4 µl 100 mmol/l Tris-HCl, 5 mmol/l CaCl₂, 22% Polyethylenglycol 6K (PEG 6K), pH 8,2 bei 4°C versetzt und gegen ein Reservoir von 500 µl 100 mmol/l Tris-HCl, 5 mmol/l CaCl₂, 22% PEG 6K, pH 8,2 bei 4°C über Dampfdiffusion im sitzenden Tropfen äquilibriert. Kristalle wuchsen nach 3-7 Tagen.

### Beispiel 16

### Test zur Auffindung von FXa Inhibitoren

FXa Proteaseinhibitoren wurden durch Inhibition der FXa Aktivität identifiziert. Dazu wurde die FXa Aktivität der rekombinant hergestellten rFXa-EGF2-AP-CD Proteasevariante in Abund Anwesenheit der zu testenden Substanz oder eines Substanzgemisches bestimmt und durch Quotientenbildung die prozentuale Hemmung errechnet. Die Hemmkonstante Ki wurde aus einer Hemmkinetik ermittelt.

### Testprinzip:

| | |
|---|---|
| Meßsignal | pNA (p-Nitroanilin) |
| FX Substrat | MOC-D-NleGlyArg-pNA (Chromozym X) |

| | |
|---|---|
| Testansatz | 200 µl Puffer |
| | + 20 µl rFXa-EGF2-AP-CD (0,13 mg/ml; 4 µmol/l) |
| | + 25 µl Substrat (Chromozym X, 8 mmol/l) |
| | + 20 µl Inhibitor |

| | |
|---|---|
| Puffer | 50 mmol/l Tris HCl, pH 7,4; 150 mmol/l NaCl; 5 mmol/l CaCl₂; 0,1% PEP |

Der Testansatz wurde in einer Mikrotiterplatte bei RT inkubiert und lineare Anfangssteigerung (ΔE/min) durch Extinktionsmessungen bei 405 nm bestimmt.

### Referenzliste

**Bang, N.U.;** Beckmann, R.J.; Jaskunas, S.R.; Lai, M.-H.T.; Little, S.P.; Long, G.L.; Santerre, R.F.: Vectors and methods for expression of human protein C activity. EP 0 191 606.

**Bergmeyer, H.U.** (ed.): Methods of Enzymatic Analysis, Vol. V, chapter 3, 3rd ed., Academic Press, New York (1983).

**Bharadwaj, D.;** Harris, R.J.; Kisiel, W.; Smith, K.J.: Enzymatic removal of sialic acid from human factor IX and factor X has no effect on their coagulant activity. J. Biol. Chem. 270, 6537-6542 (1995).

**Blow, D.M.:** Structure and mechanism of chymotrypsin. Acc. Chem. Res. 9, 145-152 (1976).

**Brinkmann, U.;** Mattes, R.E.; Buckel, P.: High-level of recombinant genes in Escherichia coli is dependent on the availability ofthe dnaY gene product. Gene 85, 109114 (1989).

**Van Dam-Mieras, M.C.E.;** Muller, A.D.; van Dieijen, G.; Hemker, H.C.: Blood coagulation factors II, V, VII, VIII, IX, X and XI: Determination with synthetic substrates. In: Bergmeyer, H.U. (ed.): Methods of Enzymatic Analysis, Vol. V, Enzymes 3: Peptidases, Proteinases and Their Inhibitors, page 365-394, 3rd ed., Academic Press, New York (1983).

**Davie, E.W.;** Fujikawa, K.; Kisiel, W.: The coagulation cascade: Initiation, maintenance, and regulation. Biochem. 30, 10363-10379 (1991).

**DiBella, E.E.;** Maurer, M.C.; Scheraga, H.A.: Expression and folding of recombinant bovine prethrombin-2 and its activation to thrombin. J. Biol. Chem. 270, 163-169 (1995).

**Esmon, C.T.,** Prothrombin activation, doctoral dissertation, Washington University, St. Louis, M0 (1973).

**Fujikawa, K.;** Legaz, M.E.; Davie, E.W.: Bovine factor XI (Stuart factor). Mechanism of activation by a protein from Russell's viper venom. Biochem. 11, 4892-4898 (1972).

**Furie, B.;** Furie, B.C.: The molecular basis of blood coagulation. Cell 53, 505-518 (1988).

**Grodberg, J.;** Dunn, J.J.: OmpT encodes the Escherichia coli outer membrane protease that cleaves T7 RNA polymerase during purification. J. Bacteriol. 170, 1245-1253 (1988).

**Hagen, F.S.;** Murray, M.J.; Busby, S.J.; Berkner, K.L.; Insley, M.Y.; Woodbury, R.G.; Gray, C.L.: Expression of factor VII activity in mammalian cells. EP 0 200 421.

**Hertzberg, M.S.;** Ben-Tal, O.; Furie, B.; Furie, B.C.: Construction, expression, and characterization of a chimera of factor IX and factor X. J. Biol. Chem. 267, 14759-14766 (1992).

**Holly, R.D.;** Foster, D.C.: Methods for producing thrombin. WO 93/13208.

**Kaul, RK.,** Hildebrand, B.; Roberts, S.; Jagadeeswaran, P.: Isolation and characterization of human blood-coagulation factor X cDNA. Gene 41, 311-314 (1986).

**Kopetzki, E.;** Rudolph, R.; Grossmann, A.: Recombinant core-streptavidin. WO 93/09144.

**Laemmli, U.K.:** Cleavage of structural proteins during the assembly of the head of bacteriophage T4. Nature 227, 680-685 (1970).

**Lin, S.-W.;** Smith, K.J.; Welsch, D.; Stafford, D.W.: Expression and characterization of human factor IX and factor IX-factor X chimeras in mouse C127 cells. J. Biol. Chem. 265, 144-150 (1990).

**McGraw, R.A.;** Davis, L.M.; Noyes, C.M.; Lundblad, R.L.; Roberts, H.R.; Graham, J.B.; Stafford, D.W.: Evidence for a prevalent dimorphism in the activation peptide of human coagulation factor IX. Proc. Natl. Acad. Sci. USA 82, 2847-2851 (1985).

**Medved, L.V.;** Orthner, C.L.; Lubon, H.; Lee, T.K.; Drohan, W.N.; Ingham, K.C.: Thermal stability and domain-domain interactions in natural and recombinant protein C. J. Biol. Chem. 270, 13652-13659 (1995).

**Mullis, K.B.;** Faloona, F.A.: Specific synthesis of DNA in vitro via a polymerase-catalyzed chain reaction. Methods Enzymol. 155, 355-350 (1987).

**Nicolaisen, E.M.;** Bjorn, S.E.; Wiberg, F.C.; Woodbury, R: Modified factor VII/VIIa. WO 88/10295

**Pedersen, A.H.;** Lund-Hansen, T.; Bisgaard-Frantzen, H.; Olsen, F.; Petersen, L.C.: Autoactivation of human recombinant coagulation factor VII. Biochem. 28, 9391-9336 (1989).

**Polgar, L.:** Structure and function of serine proteases. In: Mechanisms of protein action. Boca Raton, Florida, CRC Press, chapter 3 (1989).

**Rezaie, A.R.;** Neuenschwander, P.F.; Morrissey, J.H.; Esmon, C.T.: Analysis of the functions of the first epidermal growth factor-like domain of factor X. J. Biol. Chem. 268, 8176-8180 (1993).

**Rezaie, A.R.;** Esmon, C.T.: Asp-70-Lys mutant of factor X lacks high affinity Ca²⁺ binding site yet retains function. J. Biol. Chem. 269; 21495-21499 (1994).

**Sambrook, J.;** Fritsch, E.F.; Maniatis,T.: Molecular cloning: A laboratory manual. Cold Spring Harbor Press, Cold Spring Harbor, New York, (1989).

**Sheehan, J.P.;** Wu, Q.; Tollefsen, D.M.; Sadler, J.E.: Mutagenesis of thrombin selectively modulates inhibition by serpins heparin cofactor II and antithrombin III, J. Biol. Chem. 268, 3639-3645 (1993).

**Thogersen, H.C.;** Holtet, T.L.; Etzerodt, M.: Improved method for the refolding of proteins. WO 94/18227.

**Wolf, D.L.;** Sinha, U.; Hancock, T.E.; Lin, P.-H.; Messier, T.L.; Esmon, C.T.; Church, W.R.: Design of constructs for the expression of biologically active recombinant human factor X and Xa. J. Biol. Chem. 266, 13726-13730 (1991).

**Yee, J.;** Rezaie, A.R.; Esmon, C.T.: Glycosaminoglycan contributions to both protein C activation and thrombin inhibition involve a common arginine-rich site in thrombin that includes residues arginine 93, 97, and 101. J. Biol. Chem. 269, 17965-17970 (1994).

**Zhong, D.G.;** Smith, K.J.; Birktoft, J.J.; Bajaj, S.P.: First epidermal growth factor-like domain of human blood coagulation factor IX is required for its activation by factor VIIa tissue factor but not by factor XIa. Proc. Natl. Acad. Sci. USA 91, 3574-3578 (1994).

### SEQUENZPROTOKOLL

(1) ALLGEMEINE ANGABEN:
   (i) ANMELDER:
      (A) NAME: BOEHRINGER MANNHEIM GMBH
      (B) STRASSE: Sandhofer Str. 116
      (C) ORT: Mannheim
      (E) LAND: Deutschland
      (F) POSTLEITZAHL: D-68305
      (G) TELEFON: 08856/60-3446
      (H) TELEFAX: 08856/60-3451
   (ii) BEZEICHNUNG DER ERFINDUNG: Rekombinante Blutgerinnungsproteasen
   (iii) ANZAHL DER SEQUENZEN: 17
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30B (EPA)
(2) ANGABEN ZU SEQ ID NO: 1:
   (i) SEQUENZKENNZEICHEN:
      (A) LÄNGE: 41 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 1:
   AAAAAAGAAT TCTCATGATC GTGGGAGGCC AGGAATGCAA G                     41
(2) ANGABEN ZU SEQ ID NO: 2:
   (i) SEQUENZKENNZEICHEN:
      (A) LÄNGE: 41 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 2:
   AAAAAAAAGC TTCATTACTT GGCCTTGGGC AAGCCCCTGG T                     41
(2) ANGABEN ZU SEQ ID NO: 3:
   (i) SEQUENZKENNZEICHEN:
      (A) LÄNGE: 77 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 3:
   AATTCATTAA AGAGGAGAAA TTAAAATGCA TCACCACCAC GACGATGACG ACAAGATCGT   60
   GGGAGGCCAG GAATGCA                                                  77
(2) ANGABEN ZU SEQ ID NO: 4:
   (i) SEQUENZKENNZEICHEN:
      (A) LÄNGE: 71 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 4:
   CATTCCTGGC CTCCCACGAT CTTGTCGTCA TCGTCGTGGT GGTGATGCAT TTTAATTTCT 60
   CCTCTTTAAT G 71
(2) ANGABEN ZU SEQ ID NO: 5:
   (i) SEQUENZKENNZEICHEN:
      (A) LÄNGE: 59 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 5:
   AAAAAAGAAT TCATTAAAGA GGAGAAATTA AAATGCGGAA GCTCTGCAGC CTGGACAAC 59
(2) ANGABEN ZU SEQ ID NO: 6:
   (i) SEQUENZKENNZEICHEN:
      (A) LÄNGE: 58 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 6:
   AAAAAAGAAT TCATTAAAGA GGAGAAATTA AAATGTGCGG TAAACAGACC CTGGAACG 58
(2) ANGABEN ZU SEQ ID NO: 7:
   (i) SEQUENZKENNZEICHEN:
      (A) LÄNGE: 35 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 7:
   AAAAAACCAT GGTTGCTCAG GCTACCAGCA GCAGC 35
(2) ANGABEN ZU SEQ ID NO: 8:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 8:
   AAAAAACCAT GGTTGTTGGT GGAGAAGATG CCAAACC 37
(2) ANGABEN ZU SEQ ID NO: 9:
   (i) SEQUENZKENNZEICHEN:
      (A) LÄNGE: 42 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 9:
   AAAAAAAAGC TTCATTAAGT GAGCTTTGTT TTTTCCTTAA TC 42
(2) ANGABEN ZU SEQ ID NO: 10:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 10:
   AAAAAACCAT GGATGTAACA TGTAACATTA AGAATGGCA 39
(2) ANGABEN ZU SEQ ID NO: 11:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 11:
   GGGTTCGTCC AGTTCCAGAA GGGC 24
(2) ANGABEN ZU SEQ ID NO: 12:
   (i) SEQUENZKENNZEICHEN:
      (A) LÄNGE: 61 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 12:
   AAAAAAAGGC CTGCATTCCC ACAGGGCCCT ACCCCTGTGG AAGAGTTTCT GTTTCACAAA 60
   C 61
(2) ANGABEN ZU SEQ ID NO: 13:
   (i) SEQUENZKENNZEICHEN:
      (A) LÄNGE: 42 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 13:
   AAAAAATCCG GAAGGCAAAT AGGTGTAACG TAGCTGTTTA GC 42
(2) ANGABEN ZU SEQ ID NO: 14:
   (i) SEQUENZKENNZEICHEN:
      (A) LÄNGE: 31 Basenpaare
      (B) ART: Nucleotid
      (C) STRANGFORM: Einzelstrang
      (D) TOPOLOGIE: linear
   (ii) ART DES MOLEKÜLS: other nucleic acid
      (A) BESCHREIBUNG: /desc = "primer"
   (xi) SEQUENZBESCHREIBUNG: SEQ ID NO: 14:
   AAAAAATCCG GAGCGTGACT GGGCCGAGTC C 31
(2) ANGABEN ZU SEQ ID NO: 15:
   (i) SEQUENZKENNZEICHEN:
      (A) LÄNGE: 1404 Basenpaare
      (B) ART: Nucleotid
      (C) STRANGFORM: Einzelstrang
      (D) TOPOLOGIE: linear
   (ii) ART DES MOLEKÜLS: cDNA
   (xi) SEQUENZBESCHREIBUNG: SEQ ID NO: 15:
   CTGCTCGGGG AAAGTCTGTT CATCCGCAGG GAGCAGGCCA ACAACATCCT GGCGAGGGTC    60
   ACGAGGGCCA ATTCCTTTCT TGAAGAGATG AAGAAAGGAC ACCTCGAAAG AGAGTGCATG   120
   GAAGAGACCT GCTCATACGA AGAGGCCCGC GAGGTCTTTG AGGACAGCGA CAAGACGAAT   180
   GAATTCTGGA ATAAATACAA AGATGGCGAC CAGTGTGAGA CCAGTCCTTG CCAGAACCAG   240
   GGCAAATGTA AAGACGGCCT CGGGGAATAC ACCTGCACCT GTTTAGAAGG ATTCGAAGGC   300
   AAAAACTGTG AATTATTCAC ACGGAAGCTC TGCAGCCTGG ACAACGGGGA CTGTGACCAG   360
   TTCTGCCACG AGGAACAGAA CTCTGTGGTG TGCTCCTGCG CCCGCGGGTA CACCCTGGCT   420
   GACAACGGCA AGGCCTGCAT TCCCACAGGG CCCTACCCCT GTGGGAAACA GACCCTGGAA   480
   CGCAGGAAGA GGTCAGTGGC CCAGGCCACC AGCAGCAGCG GGGAGGCCCC TGACAGCATC   540
   ACATGGAAGC CATATGATGC AGCCGACCTG GACCCCACCG AGAACCCCTT CGACCTGCTT   600
   GACTTCAACC AGACGCAGCC TGAGAGGGGC GACAACAACC TCACCAGGAT CGTGGGAGGC   660
   CAGGAATGCA AGGACGGGGA GTGTCCCTGG CAGGCCCTGC TCATCAATGA GGAAAACGAG   720
   GGTTTCTGTG GTGGAACCAT TCTGAGCGAG TTCTACATCC TAACGGCAGC CCACTGTCTC   780
   TACCAAGCCA AGAGATTCGA AGGGGACCGG AACACGGAGC AGGAGGAGGG CGGTGAGGCG   840
   GTGCACGAGG TGGAGGTGGT CATCAAGCAC AACCGGTTCA CAAAGGAGAC CTATGACTTC   900
   GACATCGCCG TGCTCCGGCT CAAGACCCCC ATCACCTTCC GCATGAACGT GGCGCCTGCC   960
   TGCCTCCCCG AGCGTGACTG GGCCGAGTCC ACGCTGATGA CGCAGAAGAC GGGGATTGTG  1020
   AGCGGCTTCG GGCGCACCCA CGAGAAGGGC CGGCAGTCCA CCAGGCTCAA GATGCTGGAG  1080
   GTGCCCTACG TGGACCGCAA CAGCTGCAAG CTGTCCAGCA GCTTCATCAT CACCCAGAAC  1140
   ATGTTCTGTG CCGGCTACGA CACCAAGCAG GAGGATGCCT GCCAGGGGGA CAGCGGGGGC  1200
   CCGCACGTCA CCCGCTTCAA GGACACCTAC TTCGTGACAG GCATCGTCAG CTGGGGAGAG  1260
   GGCTGTGCCC GTAAGGGGAA GTACGGGATC TACACCAAGG TCACCGCCTT CCTCAAGTGG  1320
   ATCGACAGGT CCATGAAAAC CAGGGGCTTG CCCAAGGCCA AGAGCCATGC CCCGGAGGTC  1380
   ATAACGTCCT CTCCATTAAA GTGA                                          1404
(2) ANGABEN ZU SEQ ID NO: 16:
   (i) SEQUENZKENNZEICHEN:
      (A) LÄNGE: 1389 Basenpaare
      (B) ART: Nucleotid
      (C) STRANGFORM: Einzelstrang
      (D) TOPOLOGIE: linear
   (ii) ART DES MOLEKÜLS: Genom-DNA
   (xi) SEQUENZBESCHREIBUNG: SEQ ID NO: 16:
   ATGCAGCGCG TGAACATGAT CATGGCAGAA TCACCAGGCC TCATCACCAT CTGCCTTTTA   60
   GGATATCTAC TCAGTGCTGA ATGTACAGTT TTTCTTGATC ATGAAAACGC CAACAAAATT  120
   CTGAATCGGC CAAAGAGGTA TAATTCAGGT AAATTGGAAG AGTTTGTTCA AGGGAACCTT  180
   GAGAGAGAAT GTATGGAAGA AAAGTGTAGT TTTGAAGAAG CACGAGAAGT TTTTGAAAAC  240
   ACTGAAAGAA CAACTGAATT TTGGAAGCAG TATGTTGATG GAGATCAGTG TGAGTCCAAT  300
   CCATGTTTAA ATGGCGGCAG TTGCAAGGAT GACATTAATT CCTATGAATG TTGGTGTCCC  360
   TTTGGATTTG AAGGAAAGAA CTGTGAATTA GATGTAACAT GTAACATTAA GAATGGCAGA  420
   TGCGAGCAGT TTTGTAAAAA TAGTGCTGAT AACAAGGTGG TTTGCTCCTG TACTGAGGGA  480
   TATCGACTTG CAGAAAACCA GAAGTCCTGT GAACCAGCAG TGCCATTTCC ATGTGGAAGA  540
   GTTTCTGTTT CACAAACTTC TAAGCTCACC CGTGCTGAGA CTGTTTTTCC TGATGTGGAC  600
   TATGTAAATT CTACTGAAGC TGAAACCATT TTGGATAACA TCACTCAAAG CACCCAATCA  660
   TTTAATGACT TCACTCGGGT TGTTGGTGGA GAAGATGCCA AACCAGGTCA ATTCCCTTGG  720
   CAGGTTGTTT TGAATGGTAA AGTTGATGCA TTCTGTGGAG GCTCTATCGT TAATGAAAAA  780
   TGGATTGTAA CTGCTGCCCA CTGTGTTGAA ACTGGTGTTA AAATTACAGT TGTCGCAGGT  840
   GAACATAATA TTGAGGAGAC AGAACATACA GAGCAAAAGC GAAATGTGAT TCGAATTATT  900
   CCTCACCACA ACTACAATGC AGCTATTAAT AAGTACAACC ATGACATTGC CCTTCTGGAA  960
   CTGGACGAAC CCTTAGTGCT AAACAGCTAC GTTACACCTA TTTGCATTGC TGACAAGGAA 1020
   TACACGAACA TCTTCCTCAA ATTTGGATCT GGCTATGTAA GTGGCTGGGG AAGAGTCTTC 1080
   CACAAAGGGA GATCAGCTTT AGTTCTTCAG TACCTTAGAG TTCCACTTGT TGACCGAGCC 1140
   ACATGTCTTC GATCTACAAA GTTCACCATC TATAACAACA TGTTCTGTGC TGGCTTCCAT 1200
   GAAGGAGGTA GAGATTCATG TCAAGGAGAT AGTGGGGGAC CCCATGTTAC TGAAGTGGAA 1260
   GGGACCAGTT TCTTAACTGG AATTATTAGC TGGGGTGAAG AGTGTGCAAT GAAAGGCAAA 1320
   TATGGAATAT ATACCAAGGT ATCCCGGTAT GTCAACTGGA TTAAGGAAAA AACAAAGCTC 1380
   ACTTAATGA                                                        1389
(2) ANGABEN ZU SEQ ID NO: 17:
   (i) SEQUENZKENNZEICHEN:
      (A) LÄNGE: 10 Aminosäuren
      (B) ART: Aminosäure
      (C) STRANGFORM: Einzelstrang
      (D) TOPOLOGIE: linear
   (ii) ART DES MOLEKÜLS: Peptid

## Patentansprüche

1. Nicht glycosyliertes enzymatisch aktives Protein mit Serinproteaseaktivität und dessen zymogene Form bestehend aus den folgenden Domänen einer Protease der Faktor IX Familie:
a) der katalytischen Domäne, N-terminal verbunden mit
b) einer Zymogen-Aktivierungsdomäne, N-terminal verbunden mit
c) einer EGF2 Domäne.

2. Verfahren zur Herstellung eines Proteins und dessen zymogener Form gemäß Anspruch 1 durch heterologe Expression einer Nukleinsäure, welche für das genannte Protein codiert, in einem prokaryontischen Organismus durch Transformation des Organismus mit einem Expressionsvektor, der ein rekombinantes Gen enthält, welches für die genannte Protease codiert, Expression des Gens und Isolierung der so hergestellten Protease.

3. Verwendung des Proteins und dessen zymogene Form gemäß Anspruch 1 zur Identifikation von Aktivatoren und/oder Inhibitoren einer Protease aus der Faktor IX Familie.

4. Verfahren zur Bestimmung von Faktor IXa in wäßrigen Lösungen, vorzugsweise in Körperflüssigkeiten, durch Inkubation mit einem Zymogen gemäß Anspruch 1 und einem chromogenen Peptidsubstrat, wobei die Spaltung des Peptidsubstrats spezifisch durch das aktivierte Zymogen erfolgt.

## Claims

1. Non-glycosylated enzymatically-active protein having serine protease activity and its zymogenic form consisting of the following domains of a protease of the factor IX family:
a) the catalytic domain N-terminally linked to
b) a zymogen activation domain N-terminally linked to
c) an EGF2 domain.

2. Process for producing a protein and its zymogenic form as claimed in claim 1 by heterologous expression of a nucleic acid which codes for the said protein in a prokaryotic organism by transformation of the organism with an expression vector which contains a recombinant gene which codes for the said protease, expression of the gene and isolation of the protease produced in this manner.

3. Use of the protein and its zymogenic form as claimed in claim 1 to identify activators and/or inhibitors of a protease from the factor IX family.

4. Method for determining factor IXa in aqueous solutions, preferably in body fluids, by incubation with a zymogen as claimed in claim 1 and a chromogenic peptide substrate in which the peptide substrate is specifically cleaved by the activated zymogen.

## Revendications

1. Protéine enzymatiquement active, non glycosylée, avec activité de sérine protéase et sa forme zymogène, constituée par les domaines suivants d'une protéase de la famille du facteur IX :
a) le domaine catalytique, relié de manière N-terminale avec
b) un domaine d'activation de zymogène, relié de manière N-terminale avec
c) un domaine EGF2.

2. Procédé de préparation d'une protéine et de sa forme zymogène selon la revendication 1, par expression hétérologue d'un acide nucléique, qui code pour ladite protéine, dans un organisme procaryote, par transformation de l'organisme avec un vecteur d'expression, qui contient un gène recombinant, qui code pour ladite protéase, par expression du gène et par isolement de la protéase ainsi préparée.

3. Utilisation de la protéine et de sa forme zymogène selon la revendication 1, pour l'identification d'activateurs et/ou d'inhibiteurs d'une protéase de la famille du facteur IX.

4. Procédé de détermination du facteur IXa en solution aqueuse, de préférence dans des liquides corporels, par incubation avec un zymogène selon la revendication 1 et un substrat peptidique chromogène, où le clivage du substrat peptidique est réalisé de manière spécifique, par le zymogène activé.
